# EUROPEAN PATENT SPECIFICATION

(11) **EP 4 103 100 B1**
(45) Date of publication and mention of the grant of the patent: **07.05.2025**
(21) Application number: 21705073.1
(22) Date of filing: 21.01.2021
(51) Int. Cl.: A61B 90/00, A61B 34/20, A61B 17/00

(54) **DEVICES AND SYSTEMS FOR MARKING TISSUE**
VORRICHTUNGEN UND SYSTEME ZUM MARKIEREN VON GEWEBE
DISPOSITIFS ET SYSTÈMES DE MARQUAGE DE TISSU

(30) Priority: 12.02.2020 US 202062975310 P
(43) Date of publication of application: 21.12.2022
(73) Proprietor: Covidien LP, Mansfield, MA 02048 (US)
(72) Inventor: SARTOR, Joe D., Longmont, Colorado 80504 (US); WASSON, James R., Tempe, Arizona 85281 (US); CROFT, Richard L., Boulder, Colorado 80301 (US); FRUSHOUR, Scott E.M., Boulder, Colorado 80301 (US)
(74) Representative: Gray, James
(86) International application number: PCT/US2021/014307
(87) International publication number: WO 2021/162838

(56) References cited:
- EP-A1- 1 091 685
- EP-A2- 1 455 673
- US-A1- 2017 252 124

## Description

### INTRODUCTION

The disclosure relates to devices and systems for marking tissue and, more particularly, to devices and systems for marking tumors.

### BACKGROUND

In lung resection of nodules, it is becoming common practice to use localization tools to aid the surgical team in effective resection of nodules that may not be palpable with current techniques. Several preoperative and intraoperative techniques are presently being used for nodule localization. One of these techniques utilizes small fiducials that are guided to a position adjacent a lung nodule using medical imaging, such as computed tomography.
EP 1 091 685 A1 describes that lesions, biopsy sites, and other target sites in solid tissue are localized by positioning an illumination source adjacent the lesion in the solid tissue. Usually, the illumination source is positioned using a localization wire (10, 30) which is percutaneously introduced to the lesion.
US 2017/252124 A1 describes markers, probes, and related systems and methods are provided for localizing locations within a patient's body, e.g., a lesion within a breast. The marker includes an energy converter e.g., one or more photodiodes, for transforming light energy striking the marker into electrical energy, a storage device coupled to the energy converter for storing the electrical energy, a threshold element that closes a switch when the electrical energy reaches a predetermined threshold to discharge the electrical energy and cause the antenna to transmit a radio frequency (RF) signal.
EP1455673 A2 describes a system for generating a excitation field for excitation of a leadless marker assembly. One aspect of the system comprises a source generator assembly having a power supply, an energy storage device, a switching network and a source coil interconnected and configured to deliver a magnetic excitation signal waveform.

### SUMMARY

The herein claimed invention relates to a tissue marking beacon according to claim 1. Optional features are defined in the dependent claims. In accordance with an aspect of the disclosure, a tissue marking beacon includes a storage capacitor including an onboard power coil. The onboard power coil wirelessly receives energy from a radio frequency (RF) field generator and stores an electrical charge in the storage capacitor. A light-emitting diode (LED) emits pulsatile near-infrared (NIR) light upon receiving a current from the storage capacitor. A circuit is arranged between the storage capacitor and the LED. The circuit controls a flow of the current from the storage capacitor to the LED. A RF antenna receives external RF pulses and signals emission of the pulsatile NIR light from the LED in response to the external RF pulses.

In some aspects, the tissue marking beacon is configured to be implanted in a tumor. The tissue marking beacon is configured to be implanted using a syringe. The tissue marking beacon may be disposed in a titanium sleeve.

In some aspects, the circuit further includes a step-up circuit configured to increase a voltage supplied to the LED.

In some aspects, a logic circuit may control an emission pattern of the pulsatile NIR light.

In some aspects, a NIR camera detects emission of the pulsatile NIR light to determine a location of the tissue marking beacon. An emission pattern of the pulsatile NIR light is configured to be synchronized with a data capture rate and/or detector exposure (shutter) of the NIR camera.

In some aspects, a second LED emits pulsatile NIR light upon receiving a current from a second storage capacitor.

In some aspects, the emission pattern of the pulsatile NIR light from the LED is different from a second emission pattern of the pulsatile NIR light emitted from the second LED. A comparison of the emission pattern and the second emission pattern identifies a location and a depth of the tissue marking beacon.

In some aspects, a RF field generator is configured to emit RF energy.

In some aspects, the RF field generator is connected with the NIR camera. The RF field generator is configured to emit pulsatile RF energy to synchronize the emission pattern of the pulsatile NIR light with the data capture rate and/or detector exposure (shutter) of the NIR camera.

### BRIEF DESCRIPTION OF THE DRAWINGS

The accompanying drawings, which are incorporated in and constitute a part of this specification, illustrate aspects and features of the disclosure and, together with the detailed description below, serve to further explain the disclosure, in which:
FIG. 1 illustrates a tissue marking system in accordance with the disclosure;
FIG. 2 illustrates a tissue marking beacon without a sleeve in accordance with the disclosure;
FIG. 3 illustrates a tissue marking beacon with a sleeve in accordance with the disclosure;
FIGS. 4A, 4B, 4C and 4D illustrate a tissue marking beacon having at least two LEDs encapsulated in a sleeve in accordance with the disclosure;
FIG. 5 is a line graph of an exemplary current discharge to an LED in accordance with the disclosure;
FIG. 6A is a diagram of an exemplary circuit for controlling the current discharge of FIG. 5;
FIG. 6B is a diagram of an exemplary step-up circuit for increasing a current discharged by the circuit of FIG. 6A;
FIG. 6C is a line graph of an exemplary current step-up pattern of the step-up circuit of FIG. 6B;
FIG. 7A illustrates exemplary pulsatile emission patterns of NIR light in accordance with the disclosure;
FIG. 7B illustrates an exemplary circuit of an external signaling device and an exemplary circuit of a receiver in accordance with the disclosure;
FIG. 8 is a conceptual illustration of a solar photocell system including an external signaling device and a receiver of a tissue marking beacon in accordance with the disclosure;
FIGS 9A and 9B illustrate exemplary light dispersion patterns of an LED in accordance with the disclosure
FIG. 10A is a diagram of an exemplary circuit for controlling pulsatile emission patterns of at least two LEDs;
FIG. 10B is a line graph of an exemplary current discharge pattern of at least two LEDs in accordance with the disclosure; and
FIG. 11 is a block diagram of an exemplary computer of the control module of FIG. 1.

### DETAILED DESCRIPTION

As used herein, the terms parallel and perpendicular are understood to include relative configurations that are substantially parallel and substantially perpendicular up to about + or - 10 degrees from true parallel and true perpendicular.

"About" or 'approximately" or "substantially" as used herein may be inclusive of the stated value and means within an acceptable range of variation for the particular value as determined by one of ordinary skill in the art, considering tolerances (e.g., material, manufacturing, use, environmental, etc.) as well as the measurement in question and the error associated with measurement of the particular quantity (e.g., the limitations of the measurement system). For example, "about:" may mean within one or more standard variations, or within ± 30%, 20%, 10%, 5% of the stated value.

Descriptions of technical features or aspects of an exemplary embodiment of the disclosure should typically be considered as available and applicable to other similar features or aspects in another exemplary embodiment of the disclosure. Accordingly, technical features described herein according to one exemplary embodiment of the disclosure may be applicable to other exemplary embodiments of the disclosure, and thus duplicative descriptions may be omitted herein.

Exemplary embodiments of the disclosure will be described more fully below (e.g., with reference to the accompanying drawings). Like reference numerals may refer to like elements throughout the specification and drawings.

Tumor localization refers to methods of identifying the specific location of tumors in organs pre and perioperatively. In recent years, surgeons have been exploring a variety of approaches for localization, including the use of radioactive seeds, magnetic markers, and RFID. No technologies have emerged to enable the surgeon to localize a marker while actively preforming surgery as current approaches for localization involve the use of a hand-held wand to target and range the seed or implant.

Described herein is a seed (e.g., a fiducial or a beacon) with a light emitting diode (LED) powered by an inductively coupled RF antenna. As an example, an 850nm LED (e.g., having a range of from about 840nm to about 860nm) deeply penetrates tissue and can be imaged with Medtronic's Visionsense NIR imaging system^{®}. The devices and systems described herein may be employed for tumor localization as deep as 6cm (e.g., for lung cancer treatment). The LED may be configured to produce a pulsatile signal that is within the visible spectrum, and thus the LED may be identifiable intra-operatively using known white-light methods.

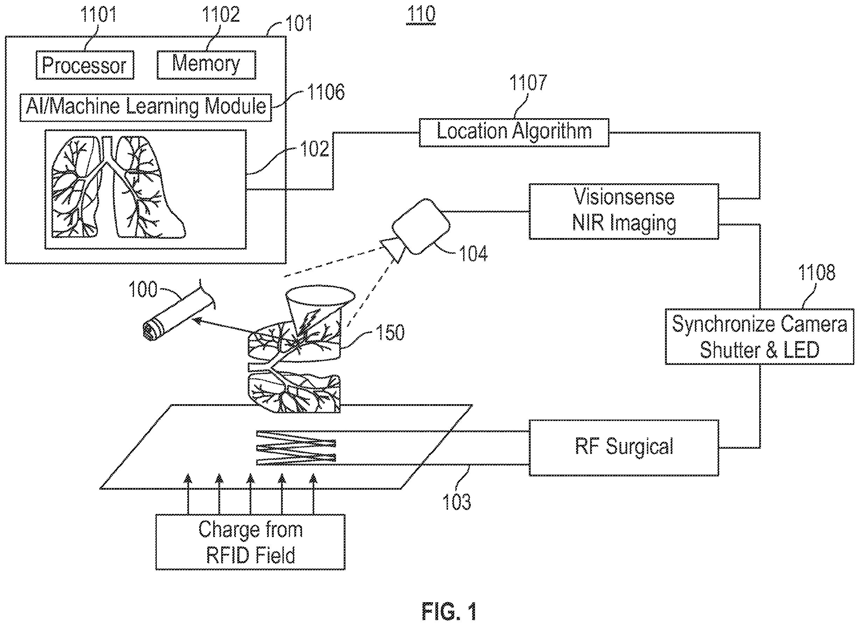

FIG. 1 illustrates a tissue marking system 110 in accordance with this disclosure. The system 110 generally includes a control module 101 having a display 102 for visualizing a position of a tissue marking beacon 100 (e.g., with respect to a surgical target such as a tumor in a patient's lung 150), a camera 104, an RF field generator 103 configured to emit RF energy, and the tissue marking beacon 100 configured to be located by the system 110. The tissue marking beacon 100 may be injected into a tumor site pre-operatively or perioperatively and then used to identify the location of the tumor.

The camera 104 is a near-infrared (NIR) camera that detects emission of pulsatile NIR light from the tissue marking beacon 100 to determine a location of the tissue marking beacon 100. An emission pattern of the pulsatile NIR light is synchronized with a data capture rate of the NIR camera 104. For example, a shutter speed/frequency of the NIR camera 104 may be synchronized with the emission pattern of the pulsatile NIR light such that the NIR light is emitted precisely when the camera shutter is in an open position, thus allowing a minimal amount of electrical current to be used by the tissue marking beacon 100.

The RF field generator 103 may be coupled with the NIR camera 104 to facilitate the synchronization between the data capture rate and/or detector exposure (shutter) of the NIR camera 104 and the emission pattern of NIR light. The RF field generator 103 is configured to emit pulsatile RF energy to control when NIR light is emitted from the tissue marking beacon 100. While tissue marking beacon 100 is illustrated in FIG. 1, any of the tissue marking beacons described herein may be employed by the tissue marking system 110.

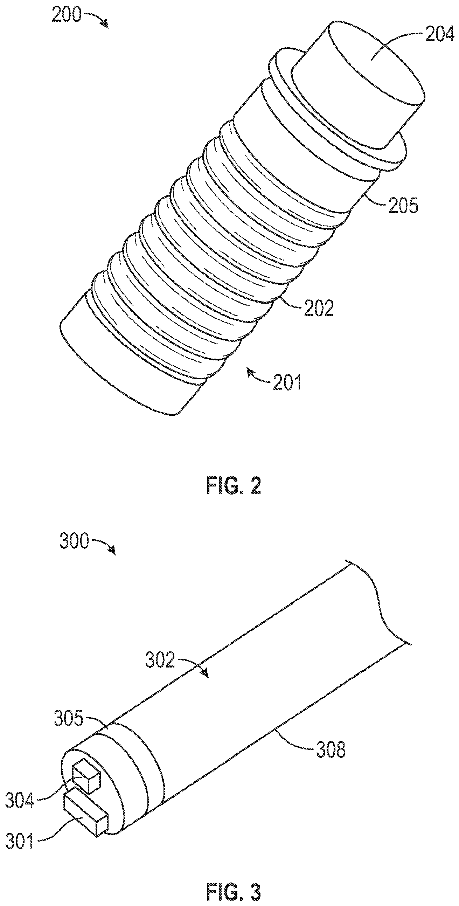

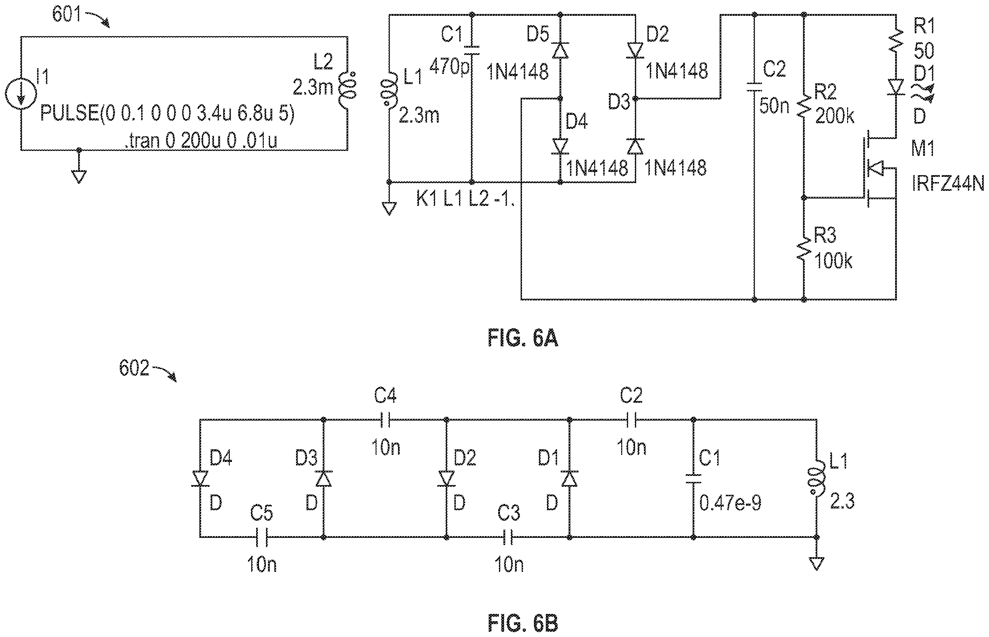

FIG. 2 illustrates a tissue marking beacon 200 in accordance with aspects of this disclosure. The tissue marking beacon 200 includes a storage capacitor 201 including an onboard power coil 202. The storage capacitor 201 is an energy storage capacitor capable of high current discharge as a ratio of total energy stored. The onboard power coil 202 wirelessly receives energy from a radio frequency (RF) field generator (see, e.g., RF filed generator 103 of FIG. 1) and stores an electrical charge in the storage capacitor 201. A light-emitting diode (LED) 204 emits pulsatile near-infrared (NIR) light upon receiving a current from the storage capacitor 201. A circuit (see, e.g., circuit 601 of FIG. 6A) is arranged between the storage capacitor 201 and the LED 204 and serves to control a flow of current from the storage capacitor 201 to the LED 204. The circuit may be configured to control a flow of current from the storage capacitor 201 to the LED 204 in a manner that alternates an emission of light from multiple LEDs. The circuit may be configured to discharge current to the LED 204 based on a signal passed through the power coil 202. The signal may be a cessation of transmitted power. An RF antenna 205 receives external RF pulses and signals emission of the pulsatile NIR light from the LED 204 in response to the external RF pulses. The RF pulses may be emitted from the RF filed generator 103. The RF field generator may be adjacent to a patient's body, or may be positioned on an instrument inside the patient's abdomen.

Each of the tissue marking beacons described herein may be dimensioned to be implanted in a tumor by a syringe (e.g., a syringe having about a 16 gauge needle). For example, the tissue marking beacon 200 is configured to be implanted in a tumor to mark a location of the tumor. The tissue marking beacons described herein can be implanted by Electromagnetic Navigation Bronchoscopy (ENB) or by an endoluminal robot.

FIG. 3 illustrates a tissue marking beacon 300 in accordance with another aspect of this disclosure. The tissue marking beacon 300 is substantially the same as the other tissue marking beacons described herein, unless otherwise indicated. The tissue marking beacon 300 includes a storage capacitor 301, an onboard power coil 302, an LED 304, and an RF antenna 305. The tissue marking beacon 300 further includes a sleeve 308. The tissue marking beacon 300 may be disposed in the sleeve 308 for injection into a tumor. The sleeve 308 may include or may be formed of titanium. Titanium is a highly durable and cost-effective material for manufacturing. Using titanium or another similar relatively low-conductivity metal reduces eddy current losses induced from the RF field generator 103. As an example, the sleeve 308 may have a wall thickness of about 0.125mm, an outer diameter of 1.25mm, and a length of from about 12mm to about 15mm.

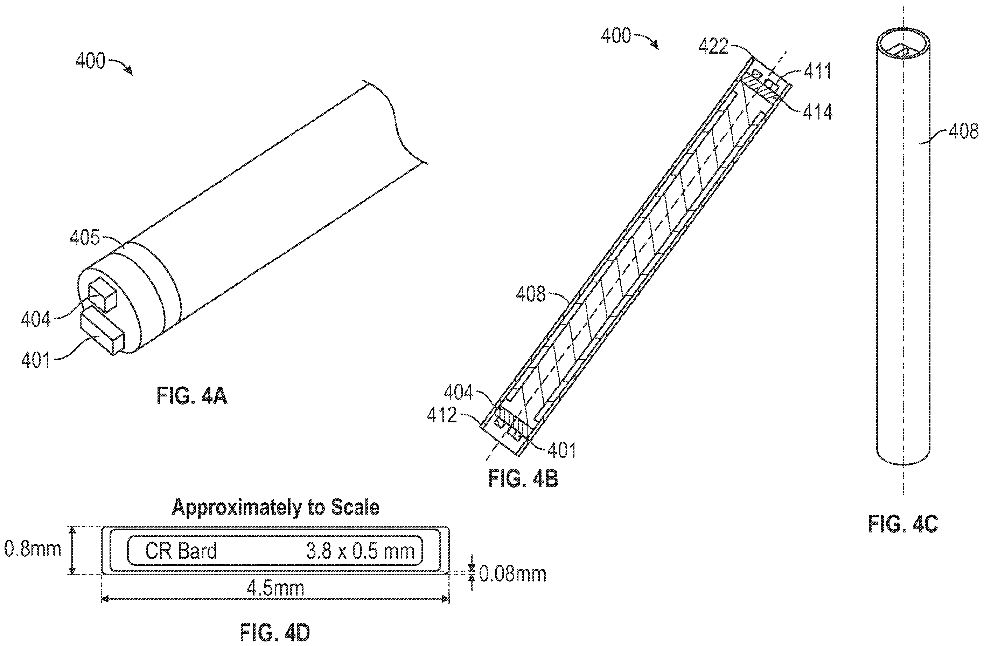

FIGS. 4A, 4B, 4C and 4D illustrate a tissue marking beacon 400 in accordance with another aspect of this disclosure having at least two LEDs. For example, tissue marking beacon 400 includes a first LED 404 and a second LED 414. An RF antenna 405 may signal NIR light emission from the first LED 404 and a corresponding RF antenna may signal NIR light emission from the second LED 414. The first LED 404 is positioned at a first end portion 412 of the tissue marking beacon 400 and the second LED 414 is positioned at a second end portion 422 of the tissue marking beacon 400 opposite the first end portion 412. The first LED 404 emits pulsatile NIR light upon receiving a current from a first storage capacitor 401. The second LED 414 emits pulsatile NIR light upon receiving a current from a second storage capacitor 411. Alternatively, a single storage capacitor may power each of the LEDs 404 and 414.

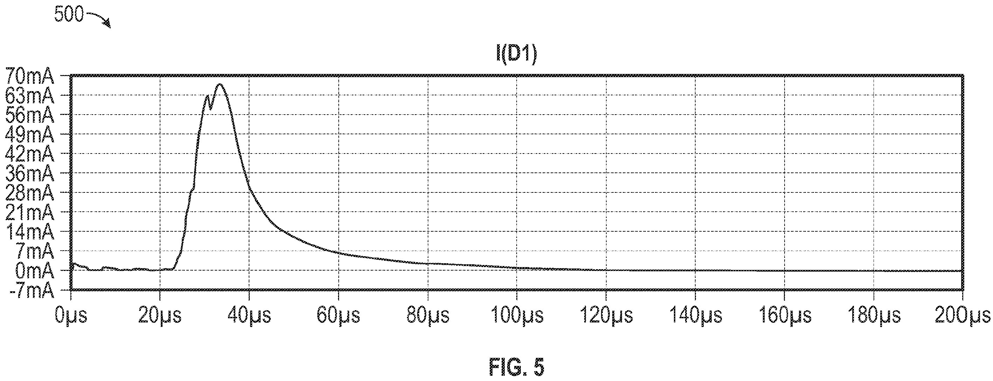

FIG. 5 is a line graph 500 of an exemplary current discharge to an LED (e.g., LEDs 204, 404 or 414). As an example, the LED 204 may emit each pulse of NIR light for about 22ms. The current discharge illustrated in FIG. 5 may be similarly applicable to other LEDs described herein, unless otherwise indicated.

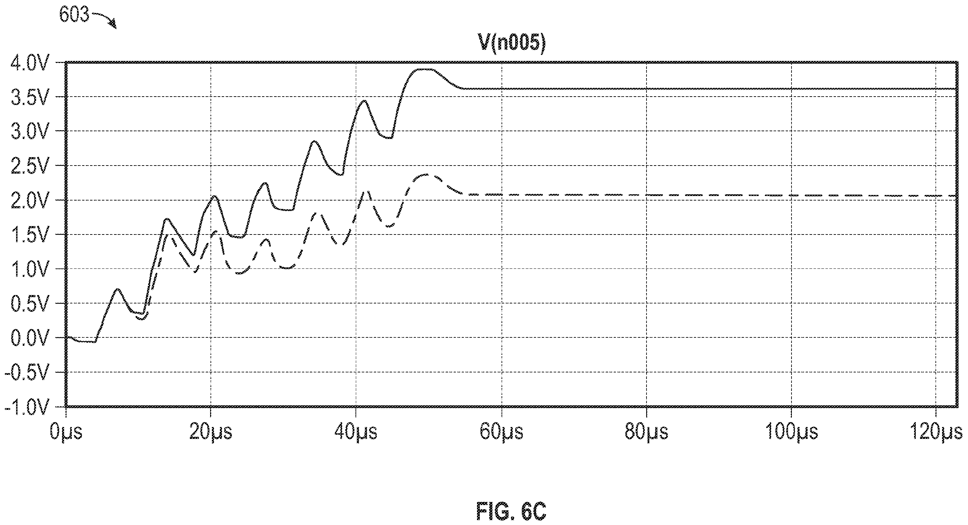

FIG. 6A is a diagram of an exemplary circuit 601 for controlling the current discharge to LEDs 204, 404 or 414. The circuit 601 may further include a step-up circuit 602 (see, e.g., FIG. 6B) configured to increase a voltage supplied to LEDs 204, 404 or 414. FIG. 6C is a line graph 603 of an exemplary current step-up pattern of the step-up circuit 602.

In some aspects, a logic circuit may control an emission pattern of the pulsatile NIR light emitted by LEDs 204, 404 or 414. The logic circuit may be onboard any of the tissue marking beacons described herein.

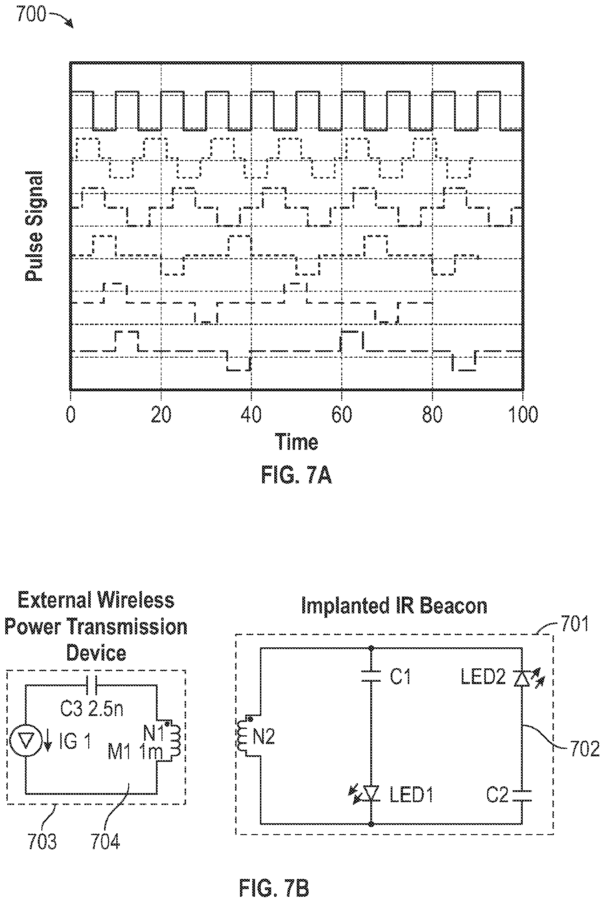

FIG. 7A displays a graph 700 illustrating exemplary pulsatile emission patterns of NIR light. As an example, each pulse of NIR light may be emitted for about 22ms. FIG. 7B illustrates an external signaling device 703 employing exemplary circuit 704 and a receiver 701 employing exemplary circuit 702.

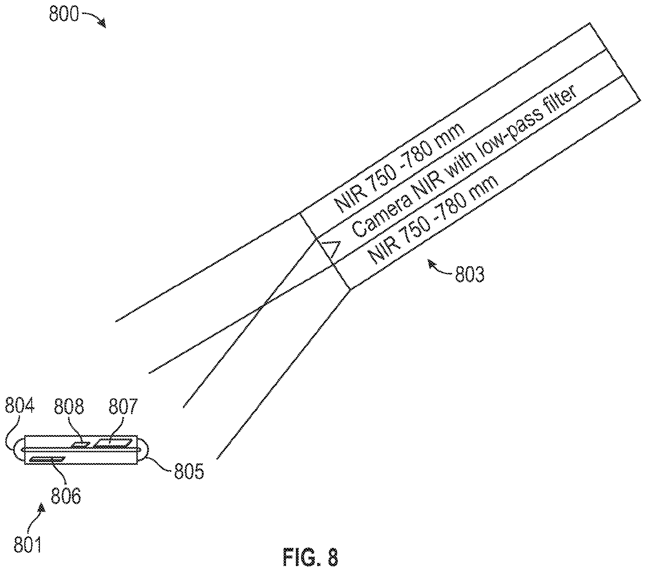

FIG. 8 is a conceptual illustration of a solar photocell system 800 in accordance with an aspect of this disclosure including an external signaling device 803 a receiver 801 operating as a tissue marking beacon. The external signaling device 803 of the solar photocell system 800 may be on board the camera (e.g., camera 104), and thus the RF pulses from the RF field generator 104 need not be used. The camera may include a low-pass filter. The external signaling device 803 may emit light signals to the receiver 801 to trigger a desired emission of pulsatile NIR light from at least one of the LEDs 804 or 805 of the receiver 801. Thus, the camera 104 need not be coupled with the RF field generator 103 to synchronize the NIR emission pattern of the tissue marking beacon with the data capture rate of the camera 104. The camera 104 may directly regulate synchronization with the tissue marking beacon. The receiver 801 includes photovoltaic power cells 807 and 808 operated by a control/storage capacitor configured to control a flow of current to the LEDs 804, 805.

The external signaling device 803 may transmit optical power at a first wavelength to the receiver 801. The receiver 801 may store electrical power (e.g., in photovoltaic power cells 807 and 808) and emit light of a second wavelength (e.g., different from the first wavelength) back to the external signaling device 803. Thus, a shutter of external signaling device 803 may be synchronized with the emitted light having the second wavelength. As an example, a signal may be transmitted in the optical power at the first wavelength to control the synchronization between the shutter of external signaling device 803 and the emitted light having the second wavelength. As an example, the external signaling device may emit light of the first wavelength in a range of from about 750nm to about 780nm and at least one of LEDs 804 or 805 may emit light of the second wavelength in a range of from about 830nm to about 850nm.

To enhance position location or detection variations a second LED/LD (Laser Diode) is added to the tissue marking beacon. For example, an LED/LD could be attached to both ends of the tissue marking beacon. The resulting two points of light can be triangulated to provide a location over distance. Two or more sources of light can also provide a relative orientation of the tissue marking beacon in free space. Each of the two LEDs may be pulsed with different identifiable emission patterns (e.g., different frequencies and/or with different emission timing). Alternatively, variations in color/wavelengths of light may be employed to differentiate opposite ends of the tissue marking beacon.

Pulsing variations of each LED can provide discrimination based upon a (known) pulse repetition rate. Increased light intensity from the LED/LD can be achieved by pulsing the current through the diode(s).

Pulsing of RF Energy to charge the RF beacon can be made synchronous to the detection camera such that the shutter and or exposure to charge the detector is synchronized with the RF pulse to the tissue marking beacon. This allows for increased discrimination of ambient light and increased signal detection accuracy.

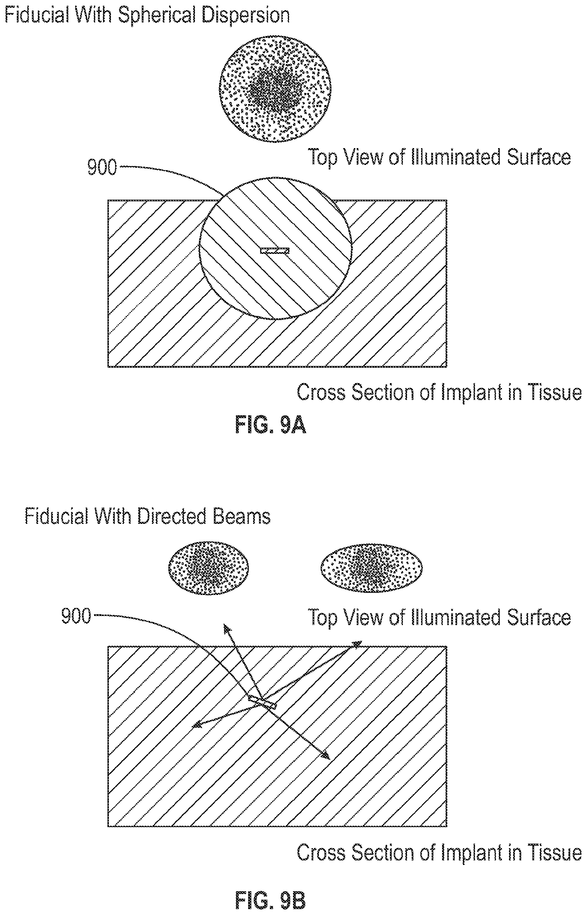

FIGS. 9A and 9B illustrate dispersion patterns of a tissue marking beacon 900 in accordance with an aspect of this disclosure. Tissue marking beacon 900 is substantially the same as tissue marking beacon 400 described herein, unless otherwise indicated. The first emission pattern of the pulsatile NIR light from the first LED is different from a second emission pattern of the pulsatile NIR light emitted from the second LED. A comparison of the first emission pattern and the second emission pattern identifies a location and a depth of the tissue marking beacon 900.

NIR light emitted from each LED may have a distinct spherical dispersion pattern. The relative intensity of the center intensity of light to the edge intensity of light allows a calculation of the depth of the tissue marking beacon 900 to be performed. This calculation may be performed because eccentricity of surface illuminations enables prediction of incident angles (see, e.g., Fig. 9B) and triangulation to estimate a depth of the tissue marking beacon 900. The size and shape of a single dispersion pattern can be employed to predict a distance from a surface of the patient's skin to the tissue marking beacon 900.

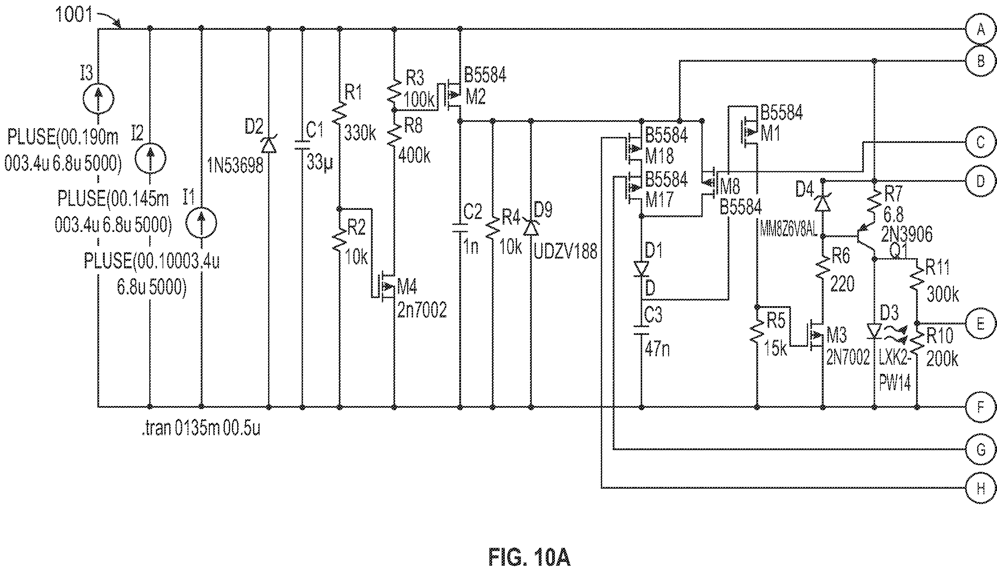

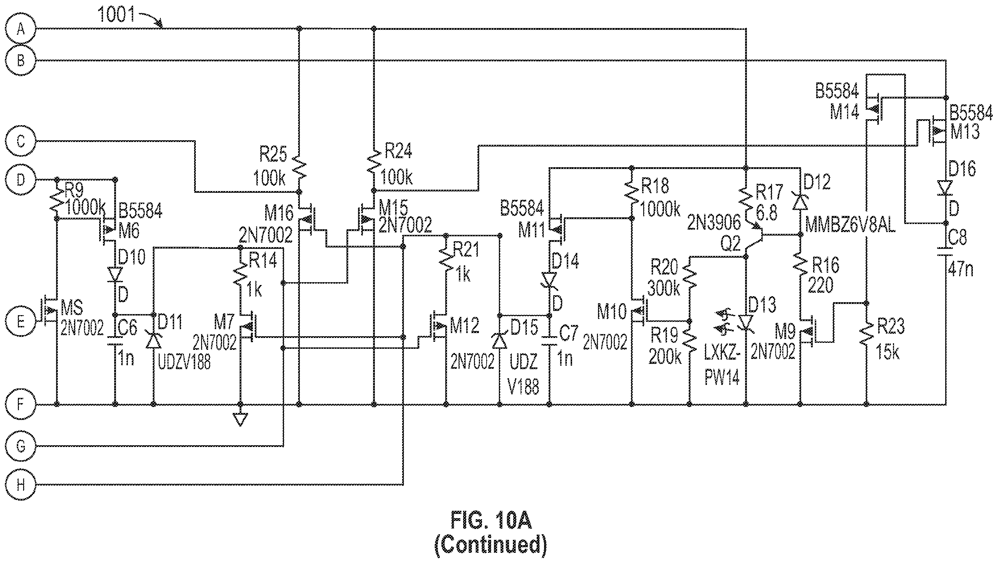

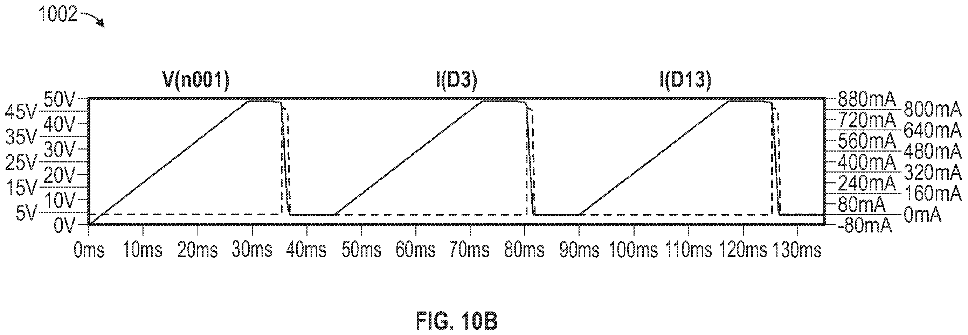

FIG. 10A is a diagram of an exemplary circuit 1001 for controlling pulsatile emission patterns of two LEDs. FIG. 10B is a line graph 1002 of an exemplary current discharge pattern of two LEDs in accordance with the disclosure.

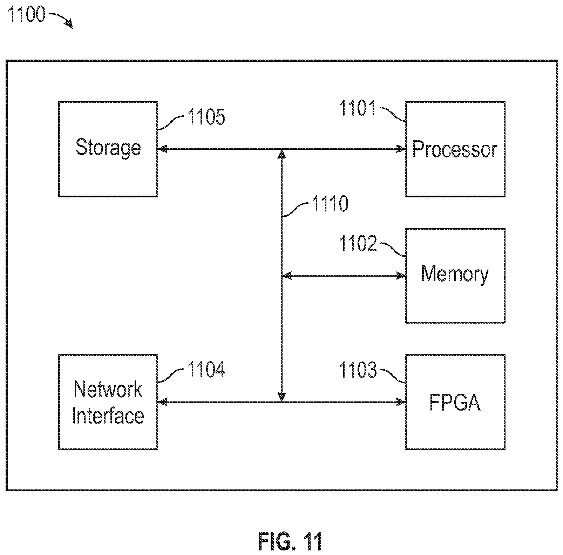

FIG. 11 is a block diagram of an exemplary computer 1100 of the control module 101 of FIG. 1. The computer of the control module 101 may include a processor 1101 connected to a computer-readable storage medium or a memory 1102 which may be a volatile type memory, *e.g.,* RAM, or a non-volatile type memory, *e.g.,* flash media, disk media, *etc.* The processor 1101 may be another type of processor such as, without limitation, a digital signal processor, a microprocessor, an ASIC, a graphics processing unit (GPU), field-programmable gate array (FPGA), or a central processing unit (CPU).

The memory 1102 can be random access memory, read-only memory, magnetic disk memory, solid state memory, optical disc memory, and/or another type of memory. The memory 1102 can communicate with the processor 1101 through communication buses 1110 of a circuit board and/or through communication cables such as serial ATA cables or other types of cables. The memory 1102 includes computer-readable instructions that are executable by the processor 1101 to operate the control module. The control module 101 may include a network interface 1104 to communicate with other computers or a server. A storage device 1105 may be used for storing data. The control module 101 may include an AI or machine learning module 1106 (see, FIG. 1) including one or more FPGAs 1103. The FPGAs 1103 may be used for executing various machine learning algorithms such as those described herein (e.g., a location algorithm 1107 or a camera shutter/LED synchronization algorithm 1108).

The storage device 1105 of the control module 101 store one or more machine learning algorithms and/or models, configured to determine a location, depth, and/or directional orientation of at least one tissue locating beacon, and to render an image on the display 102 of the tissue locating beacon with respect to a patient's anatomy. The machine learning algorithm may apply mathematical models to determine a location, depth, and/or directional orientation of at least one tissue locating beacon, and to render an image on the display 102 of the tissue locating beacon with respect to a patient's anatomy. The machine learning algorithm(s) may be trained on and learn from experimental data and/or data from previous procedures initially input into the one or more machine learning applications in order to enable the machine learning application(s) to determine a location, depth, and/or directional orientation of at least one tissue locating beacon, and to render an image on the display 102 of the tissue locating beacon with respect to a patient's anatomy.

Machine learning algorithms are advantageous for use in determining a location, depth, and/or directional orientation of at least one tissue locating beacon, and to render an image on the display 102 of the tissue locating beacon with respect to a patient's anatomy, at least in that complex sensor components and pre-defined categorization rules and/or algorithms are not required. Rather, machine learning algorithms utilize initially input data to determine statistical features and/or correlations by analyzing data therefrom. Thus, with the one or more machine learning algorithms having been trained as detailed above, such can be used to determine a location, depth, and/or directional orientation of at least one tissue locating beacon, and to render an image on the display 102 of the tissue locating beacon with respect to a patient's anatomy.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

## Claims

1. A tissue marking beacon (400), comprising:
a storage capacitor (201, 401) including an onboard power coil (202), the onboard power coil configured to wirelessly receive energy from a radio frequency (RF) field generator (103) and store an electrical charge in the storage capacitor;
wherein the tissue marking beacon comprises at least one first light-emitting diode (LED) (404) configured to emit pulsatile near-infrared (NIR) light upon receiving a current from the storage capacitor; and
a second storage capacitor (411); and
at least one second LED (414).

2. The tissue marking beacon (400) of claim 1, wherein the tissue marking beacon is configured to be implanted in a tumor.

3. The tissue marking beacon (400) of claim 2, wherein the tissue marking beacon is configured to be implanted using a syringe.

4. The tissue marking beacon (400) of claim 1, wherein the tissue marking beacon is disposed within a titanium sleeve (308).

5. The tissue marking beacon (400) of claim 1, wherein the tissue marking beacon is disposed within a glass sleeve.

6. The tissue marking beacon (400) of claim 1, wherein the emitted NIR light is observed by a NIR camera (104).

## Patentansprüche

1. Gewebemarkierungsleuchtfeuer (Beacon, 400), umfassend:
einen Speicherkondensator (201, 401) mit einer integrierten Leistungsspule (202), wobei die integrierte Leistungsspule dazu ausgelegt ist, Energie von einem Funkfrequenz (RF)-Feldgenerator (103) drahtlos zu empfangen und eine elektrische Ladung in dem Speicherkondensator zu speichern;
wobei das Gewebemarkierungsleuchtfeuer wenigstens eine erste Leuchtdiode (LED) (404) umfasst, die dazu ausgelegt ist, bei Empfang eines Stroms von dem Speicherkondensator pulsierendes Nah-Infrarot (NIR)-Licht zu emittieren; und
einen zweiten Speicherkondensator(411); und
wenigstens eine zweite LED (414).

2. Gewebemarkierungsleuchtfeuer (400) nach Anspruch 1, wobei das Gewebemarkierungsleuchtfeuer dazu ausgelegt ist, in einen Tumor implantiert zu werden.

3. Gewebemarkierungsleuchtfeuer (400) nach Anspruch 2, wobei das Gewebemarkierungsleuchtfeuer dazu ausgelegt ist, unter Verwendung einer Spritze implantiert zu werden.

4. Gewebemarkierungsleuchtfeuer (400) nach Anspruch 1, wobei das Gewebemarkierungsleuchtfeuer in einer Titanhülse (308) angeordnet ist.

5. Gewebemarkierungsleuchtfeuer (400) nach Anspruch 1, wobei das Gewebemarkierungsleuchtfeuer in einer Glashülse angeordnet ist.

6. Gewebemarkierungsleuchtfeuer (400) nach Anspruch 1, wobei das emittierte NIR-Licht von einer NIR-Kamera (104) beobachtet wird.

## Revendications

1. Balise de marquage de tissu (400), comprenant :
un condensateur de stockage (201, 401) comprenant une bobine d'alimentation embarquée (202), la bobine d'alimentation embarquée étant configurée pour recevoir sans fil l'énergie d'un générateur de champ radiofréquence (RF) (103) et stocker une charge électrique dans le condensateur de stockage ;
la balise de marquage de tissu comprenant au moins une première diode électroluminescente (DEL) (404) configurée pour émettre une lumière pulsatile dans le proche infrarouge (NIR) lorsqu'elle reçoit un courant du condensateur de stockage ; et
un deuxième condensateur de stockage (411) ; et
au moins une deuxième DEL (414).

2. Balise de marquage de tissu (400) selon la revendication 1, la balise de marquage de tissu étant configurée pour être implantée dans une tumeur.

3. Balise de marquage de tissu (400) selon la revendication 2, la balise de marquage de tissu étant configurée pour être implantée à l'aide d'une seringue.

4. Balise de marquage de tissu (400) selon la revendication 1, la balise de marquage de tissu étant disposée à l'intérieur d'un manchon en titane (308).

5. Balise de marquage de tissu (400) selon la revendication 1, la balise de marquage de tissu étant disposée à l'intérieur d'un manchon en verre.

6. Balise de marquage de tissu (400) selon la revendication 1, la lumière NIR émise étant observée par une caméra NIR (104).
